Europäisches Patentamt

European Patent Office

Office européen des brevets

(19)

(11) **EP 0 369 177 B1**

(12) **EUROPÄISCHE PATENTSCHRIFT**

(45) Veröffentlichungstag und Bekanntmachung des Hinweises auf die Patenterteilung:
**02.01.1997 Patentblatt 1997/01**

(51) Int. Cl.$^6$: **A61B 17/225**, B06B 1/00

(21) Anmeldenummer: 89119294.0

(22) Anmeldetag: **17.10.1989**

(54) **Vorrichtung zur Erzeugung von fokussierten akustischen Druckwellen**

Focused acoustic pressure wave generator

Dispositif pour générer des ondes de choc acoustiques focalisées

(84) Benannte Vertragsstaaten:
**AT BE CH DE ES FR GB IT LI LU NL SE**

(30) Priorität: **17.10.1988 DE 3835318**

(43) Veröffentlichungstag der Anmeldung:
**23.05.1990 Patentblatt 1990/21**

(73) Patentinhaber: **STORZ MEDICAL AG**
**CH-8280 Kreuzlingen (CH)**

(72) Erfinder:
• **Wess, Othmar, Dr.**
**CH-8574 Lengwill-Oberhofen (CH)**
• **Marlinghaus, Ernst H., Dr.**
**CH-8598 Bottighofen (CH)**

(74) Vertreter: **Münich, Wilhelm, Dr. et al**
**Kanzlei Münich, Steinmann, Schiller**
**Wilhelm-Mayr-Str. 11**
**80689 München (DE)**

(56) Entgegenhaltungen:
**EP-A- 0 108 190**  **EP-A- 0 188 750**
**EP-A- 0 251 797**  **EP-A- 0 265 742**
**DE-A- 3 119 295**  **SU-A- 1 393 489**
**US-A- 3 587 038**

Bemerkungen:
Die Akte enthält technische Angaben, die nach dem Eingang der Anmeldung eingereicht wurden und die nicht in dieser Patentschrift enthalten sind.

## Beschreibung

### Technisches Gebiet

Die Erfindung bezieht sich auf eine Vorrichtung zur Erzeugung von fokussierten akustischen Druckwellen für therapeutische Anwendungen und insbesondere zur Zerstörung von Konkrementen, Körpersteinen etc. gemäß dem Oberbegriff des Patentanspruchs 1.

### Stand der Technik

Eine derartige Vorrichtung ist aus der SU 1393-489-A bekannt. Bei dieser gattungsgemäßen Vorrichtung ist eine Schallerzeugungseinheit vorhanden, die Druckwellen erzeugt, die ein Reflektor mit paraboloider Kontur fokussiert. Dabei ist die sich in Richtung der Achse erstrekkende (innere) Mantelfläche des Körpers die Abstrahlfläche für die Schallwellen, so daß die Schallerzeugungseinheit (nach innen gerichtete) axialsymmetrische Druckwellen erzeugt, deren Ausbreitungsrichtung im wesentlichen senkrecht zu dieser Achse ist.

Weitere ähnliche Vorrichtungen sind beispielsweise aus den deutschen Offenlegungsschriften 34 47 440, 31 19 295, 35 05 894, 35 02 751, 35 01 838 oder 34 43 383 bekannt und werden beispielsweise zur extrakorporalen Lithotripsie verwendet.

Die extrakorporale Lithotripsie und andere therapeutische Anwendungen von akustischen Wellenfeldern, d.h. von Pulsschall- oder Stoßwellen, basieren wesentlich darauf, daß hochenergetische Therapiewellen mit geringer Energiedichte in den Körper eingekoppelt und durch Fokussierung in einem eng umgrenzten Wirkungsareal, nämlich der Fokuszone des fokussierten Wellenfelds therapeutisch wirksam werden.

Damit sind bei Vorrichtungen zur Erzeugung von fokussierten akustischen Wellenfeldern für therapeutische Anwendungen drei Probleme zu lösen:

1. Die Erzeugung einer hochenergetischen Therapiewelle mit geringer Energiedichte bei möglichst großem Wirkungsgrad,

2. die Fokussierung dieser Therapiewelle in einen möglichst kleinen Volumenbereich

Der Fokussierungsgrad der Therapiewelle wird durch das zeitliche und räumliche Wellenprofil sowie den Öffnungswinkel (Apertur) des Wellenfeldes bestimmt; die Apertur wiederum bestimmt die Energiedichte des Therapiefeldes an der Körpereintrittsstelle sowie die Energiedichte in der Fokuszone.

Diese Parameter wiederum beeinflussen die Schmerzwirkung und den Therapieerfolg sowie die Eingrenzung der Therapiewirkung auf den gewünschten Bereich.

Bei den bekannten Vorrichtungen zur Erzeugung von fokussierten akustischen Wellenfeldern für therapeutische Anwendungen, die insbesondere zur extrakorporalen Nieren- und Gallenstein-Therapie dienen, finden im wesentlichen drei verschiedene Erzeugungs- und Fokussierungsprinzipien Anwendung:

- die elektromagnetische Druckwellenerzeugung,

- die piezo-elektrische Druckwellenerzeugung,

- die elektrohydraulische Stoßwellenerzeugung.

Diese Prinzipien zur Erzeugung von Wellenfeldern besitzen unterschiedliche Daten bezüglich Effektivität, Standzeit, Verbrauchsmaterialien, Fokusgröße, Energiedichte etc..

Keines der genannten Prinzipien ist in der Lage, sämtliche Anforderungen zufriedenstellend abzudekken. Aus diesem Grunde konnte sich bislang keines der genannten Prinzipien gegenüber den anderen eindeutig durchsetzen:

Beispielsweise hat die elektromagnetische Druckwellenerzeugung den Nachteil, daß hierbei ebene Wellen erzeugt werden, die durch akustische Linsen oder - hierzu wird auf die DE 34 47 440 A1 oder die DE 35 01 838 A1 verwiesen - nach Umlenkung an einem Reflektor ohne fokussierende Wirkung mittels eines fokussierenden Reflektors sowie gegebenenfalls zusätzlichen Linsen fokussiert werden müssen.

Akustische Linsen ausreichender Qualität sind jedoch gegenwärtig zumindest in der Praxis nicht realisierbar, so daß die resultierende Therapiefeldverteilung hinsichtlich Fokussierungsgrad, Aperturwinkel und Größe der Fokuszone anderen Verfahren unterlegen ist, die ihrerseits in anderen wesentlichen Punkten, wie beispielsweise der Lebensdauer des Schallerzeugers unbefriedigend sind.

Darüberhinaus ist es bei den bekannten Vorrichtungen zur Erzeugung von fokussierten akustischen Wellenfeldern schwierig, eine Ortungseinheit beispielsweise für Nierensteine im Bereich der Vorrichtung anzuordnen, ohne daß durch die Ortungseinheit das therapeutische Wellenfeld gestört wird.

### Darstellung der Erfindung

Der Erfindung liegt die Aufgabe zugrunde, eine Vorrichtung zur Erzeugung von fokussierten akustischen Wellenfeldern für therapeutische Anwendungen und insbesondere zur Zerstörung von Konkrementen, Körpersteinen etc. anzugeben, bei der das fokussierte therapeutische Wellenfeld eine große Apertur und eine optimierte Fokussierungszone hat, ohne daß bei der Lebensdauer der Schallerzeugungseinheit Einschränkungen hingenommen werden müßten, oder der Einbau einer Ortungseinheit für Körpersteine etc. erschwert wäre.

Eine erfindungsgemäße Lösung dieser Aufgabe ist mit ihren Weiterbildungen in den Patentansprüchen gekennzeichnet.

Die Erfindung geht von dem Grundgedanken aus, eine flächenförmige Schallerzeugungseinheit, die ein nichtfokussiertes Wellenfeld abstrahlt, dessen Energiedichte mit zunehmendem Abstand von der Schallerzeugungseinheit abnimmt, mit einem Reflektor für das Wellenfeld zu kombinieren, der das Wellenfeld fokussiert.

Reflektoren für Wellenfelder sind zwar seit langem bekannt, für Vorrichtungen zur Erzeugung von fokussierten akustischen Wellenfeldern für therapeutische Anwendungen sind sie jedoch bislang lediglich zur Fokussierung von Stoßwellen, die an einer Funkenstrecke erzeugt werden, oder zur Fokussierung von ebenen Wellenfeldern, die an einem nicht fokussierenden Reflektor reflektiert worden sind, verwendet worden. Hierzu wird beispielsweise auf die DE-PS 32 41 026 oder die DE 34 47 440 A1 verwiesen.

Die Verwendung von Funkenstrecken zur Stoßwellenerzeugung hat jedoch den Nachteil, daß ein "quasi punktförmiges" Wellenfeld erzeugt wird, das - wenn es für therapeutische Anwendungen geeignet sein soll - eine sehr hohe Energiedichte haben muß, die abträglich für die Lebensdauer der Vorrichtung ist. Andererseits hat die in der DE 34 47 440 A1 oder in der DE 35 01 838 A1 beschriebene Anordnung den Nachteil, daß der das ebene Wellenfeld umlenkende Reflektor die Anordnung von anderen Bauteilen, wie beispielsweise Ortungseinheiten für Konkremente, in der Achse der Vorrichtung verhindert. Darüberhinaus ist auch bei einer derartigen Anordnung die Energiedichte an der vergleichsweise kleinen Abstrahlfläche der Schallerzeugungseinheit hoch, was zu einer hohen Belastung der Schallerzeugungseinheit sowie des nicht fokussierenden Reflektors führt.

Erfindungsgemäß wird eine Schallerzeugungseinheit entsprechend dem Oberbegriff des Anspruchs 1 verwendet, die zentral angeordnet ist und deren äußere Mantelfläche die Abstrahlfläche für die Druckwellen ist, so daß ein im wesentlichen senkrecht von der durch den Fokuspunkt gehenden Achse weggerichtetes Wellenfeld erzeugt wird. Erfindungsgemäß wird ferner ein Reflektor verwendet, der zur Fokussierung der Druckwellen eine paraboloide Kontur aufweist und der die Schallerzeugungseinheit umgibt. Durch diese Anordnung ist die Energiedichte im Bereich der Schallerzeugungseinheit gering, so daß die Lebensdauer der erfindungsgemäßen Vorrichtung nicht durch die Abnutzung der Schallerzeugungseinheit zu stark begrenzt wird.

Die Verwendung eines akustischen Reflektors hat gegenüber der Verwendung von akustischen Linsen den Vorteil, daß akustische Reflektoren mit hohem Wirkungsgrad verfügbar sind, die das akustische Wellenfeld mit nur geringen Verlusten fokussieren.

Vor allem aber hat die angegebene Konfiguration, bei der die Abstrahlfläche symmetrisch zu einer durch den Fokuspunkt gehenden Achse ausgebildet ist, den Vorteil, daß das fokussierte akustische Wellenfeld eine große Apertur aufweist. Zudem wird ein fokussiertes akustisches Wellenfeld lediglich außerhalb eines die durch den Fokuspunkt gehende Achse beispielsweise kegelförmig umgebenden Bereichs erzeugt. In dem von dem akustischen Therapie-Wellenfeld freien Bereich, d.h. innerhalb der die durch den Fokuspunkt gehenden Achse rotationssymmetrisch umgebenden Abstrahlfläche der Schallerzeugungseinheit, kann damit ohne Schwierigkeiten und insbesondere ohne das akustische Wellenfeld zu stören die Ortungseinheit für Körpersteine etc. angeordnet werden (Anspruch 7), die beispielsweise gemäß Anspruch 8 eine Röntgeneinheit oder gemäß Anspruch 9 eine Ultraschall-Ortungseinheit sein kann.

Dabei ist es besonders vorteilhaft, daß es die erfindungsgemäße Konfiguration ohne Schwierigkeiten die Verwendung eines Hohlkörpers ermöglicht, so daß die Ortungseinheit nicht nur in bzw. an der Schallerzeugungseinheit angeordnet, sondern auch längs der Achse der Vorrichtung verschoben und/oder um diese Achse gedreht (Anspruch 10) werden kann. Damit kann die Ortungseinheit Körpersteine ohne Schwierigkeiten und ohne durch Beugung auftretende Fehler erfassen.

Im Anspruch 2 sind bevorzugte Ausführungsformen für die Abstrahlfläche der Schallerzeugungseinheit angegeben, die insbesondere den Vorteil haben, daß sie ein Wellenfeld mit einer zeitlichen Verteilung erzeugen, die phasenrichtig, d.h. mit hohem Wirkungsgrad in einem kleinen Volumenbereich fokussierbar ist.

Im Anspruch 3 ist eine mögliche Reflektorform angegeben, gemäß dem der Reflektor die Form eines Rotationsparaboloids hat, das die Schallerzeugungseinheit im Bereich der durch den Fokuspunkt gehenden Achse durchsetzt.

Ausdrücklich wird jedoch darauf hingewiesen, daß auch andere Formen von Reflektoren, wie beispielsweise von Reflektoren, die aus einzelnen Segmenten, die untereinander unterschiedliche Form haben und jeweils symmetrisch zur Achse sind, verwendet werden können.

Ferner ist es möglich, die Reflektoroberfläche so auszubilden, daß erzeugte Oberflächenwellen nicht mit reflektierten Wellen interferieren. Hierzu wird auf die eingangs genannte DE-PS 32 41 026 verwiesen.

Innerhalb des erfindungsgemäßen Grundgedankens, eine "flächige" Schallerzeugungseinheit zu verwenden, die ein zur durch den Fokuspunkt gehenden Achse symmetrisches Wellenfeld erzeugt, das im wesentlichen senkrecht von dieser Achse weggerichtet ist, können selbstverständlich die verschiedensten Prinzipien der Schallerzeugung verwendet werden, solange diese nur auf der Mantelflache der Schallerzeugungseinheit radial abgestrahlte Wellenfelder erzeugen können:

Beispielsweise ist es gemäß den Ansprüchen 5 bzw. 6 möglich, auf der Abstrahlfläche der Schallerzeugungseinheit Piezo-Schwinger vorzusehen, die das Schallfeld erzeugen, oder einen radial polarisierten Piezokeramik-Hohlzylinder zu verwenden.

Besonders vorteilhaft ist es jedoch, gemäß Anspruch 4 eine elektromagnetische Druckwellenerzeugungseinheit zu verwenden. In den Ansprüchen 15f. sind Ausbildungen dieser elektromagnetischen Druckwellenerzeugungseinheit beschrieben, die mit hohem Wirkungsgrad und großer Lebensdauer ein therapeutisches Schallfeld erzeugen:

Insbesondere kann die Spule zur Energieerhöhung aus mehreren ineinander angeordneten Einzelspulen bestehen, die elektrisch parallel geschaltet sind. Dabei ist es bevorzugt, wenn zwischen Spule und Membran eine Isolierschicht angeordnet ist, die insbesondere zur Verbesserung der elektrischen Sicherheit beitragt.

Gemäß der im Anspruch 17 gekennzeichneten Ausführungsform, steht die Membran aus einem elektrisch hochleitfähigem Material, wie beispielsweise Gold, Silber, Kupfer oder Legierungen hiervon. In Anspruchen 18 ist gekennzeichnet, daß das feste Stützmaterial elektrisch von der Membran isoliert ist. Im Anspruch 19 sind vorteilhafte Materialwahlmöglichkeiten für das Stützmaterial angegeben, das insbesondere Edelstahl, Titan oder Beryllium sein kann.

Die im Anspruch 21 angegebene Weiterbildung, gemäß der das Stützmaterial fest mit der Membran verbunden ist, verbessert die Schall-Abstrahleigenschften.

Dies gilt auch für die Ausbildungen gemäß den Ansprüchen 22 bis 25

Durch die im Anspruch 22 gekennzeichnete Ausbildung, bei der die Membran und/oder das Stützmaterial keine konstante Dicke haben, läßt sich das erzeugte Schallfeld in gewissen Grenzen wunschgemäß einstellen.

Vorteilhafterweise weist die Schallerzeugungseinheit zur Erzeugung von Schallimpulsen einen Piezoschwinger auf, der -wie eingangs dargelegt, insbesondere im Hinblick auf das generelle Problem, eine hochenergetische Therapiewelle mit geringer Energiedichte im Bereich der Abstrahlfläche bei möglichst großem Wirkungsgrad zu erzeugen, weitergebildet ist.

Die erfindungsgemäße Vorrichtung kann selbstverständlich für Lithotripter verwendet werden, die mit einer Patientenwanne arbeiten, wie sie in der Literatur beschrieben ist. Da die erfindungsgemäße Vorrichtung jedoch sehr klein und kompakt ist, kann sie in besonders vorteilhafter Weise in Lithotriptern eingesetzt werden, die "Wannen-frei" arbeiten. Hierbei ist es besonders vorteilhaft, wenn der Reflektor von einer schalldurchlässig und akustisch angepaßten Membran abgeschlossen ist (Anspruch 12) und das Koppelmedium in an sich bekannter Weise ein Medium mit gewebeähnlichen akustischen Eigenschaften und insbesondere eine Flüssigkeit ist (Ansprüche 13 bzw. 14).

## Kurze Beschreibung der Zeichnung

Die Erfindung wird nachstehend anhand von Ausführungsbeispielen unter Bezugnahme auf die Zeichnung näher beschrieben, in der zeigen:

Fig. 1 ein Ausführungsbeispiel einer erfindungsgemäßen Vorrichtung zur Erzeugung von fokussierten akustischen Wellenfeldern,

Fig. 2 bis 4 verschiedene Möglichkeiten der Integration von Ortungseinheiten in die in Figur 1 dargestellte Vorrichtung, und

Fig. 5 a und b zwei mögliche Ausführungen für die Schallerzeugungseinheit.

## Darstellung von Ausführungsbeispielen

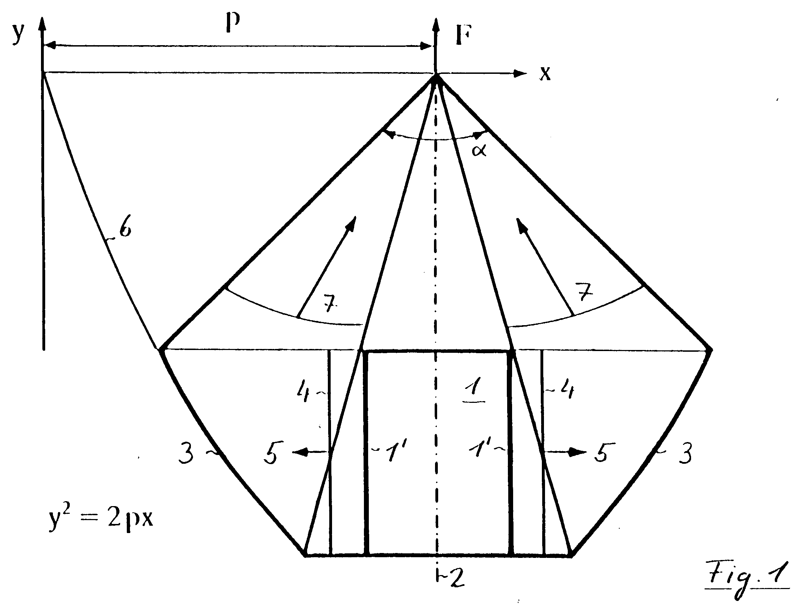

Figur 1 zeigt ein Ausführungsbeispiel einer erfindungsgemäßen Vorrichtung zur Erzeugung von fokussierten akustischen Wellenfeldern, das eine Schallerzeugungseinheit 1 für das Therapiewellenfeld aufweist, deren Abstrahlfläche 1′ bei dem gezeigten Ausführungsbeispiel die Form eines Zylinders hat. Koaxial zur Achse 2 der Vorrichtung, die mit der Achse der zylinderförmigen Abstrahlfläche 1′ zusammenfällt, ist ein rotationssymmetrischer Reflektor 3 angeordnet, dessen Innenfläche in jedem Schnitt die Form einer Parabel hat.

Da die Schallerzeugungseinheit - wie nachfolgend noch erläutert werden wird - zylindrische Wellenfronten 4 erzeugt, die sich in Radialrichtung 5 von der Schallerzeugungseinheit 1 wegbewegen, ergeben sich nach Reflexion an dem Reflektor 3 mit paraboloider Innenkontur fokussierte Wellenfelder 7 mit dem Aperturwinkel α, die im Fokuspunkt F zusammenlaufen und dort Leistungsdichten erzeugen, die therapeutisch wirksam sind.

Die Parabel 6, die sich in jedem die Achse 2 enthaltenden Schnitts des Reflektors 3 ergibt, ist durch die folgende Gleichung gegeben:

$$Y^2 = 2px$$

wobei p der Abstand des Brennpunktes F vom Koordinatenursprung ist; die Lage des verwendeten Koordinatensystems ist Figur 1 zu entnehmen.

Ausdrücklich soll darauf hingewiesen werden, daß bei sehr hohen Amplituden der akustischen Therapiewellen deren Ausbreitungsverhalten vom linearen Ausbreitungsverhalten abweichen kann, so daß sich Veränderungen der Fokusgeometrie ergeben können; diese Veränderungen der Fokusgeometrie können durch entsprechende Anpassung der Reflektorfläche, beispielsweise durch geringfügige Abweichungen von der Parabelform kompensiert werden.

Ebenso können gezielte Änderungen des fokussierten Therapiewellenfeldes durch definierte Abweichungen von der Parabelform und/oder durch eine entsprechende Änderung des Reflexionsverhaltens der Oberfläche erreicht werden.

Je nachdem ob man eine Reflexion der Welle mit oder ohne Phasenumkehr anstrebt, wählt man ein Material für den Reflektor, das akustisch härter bzw. akustisch weicher ist als die Koppelflüssigkeit. Im Fall der Erzeugung von vorwiegenden positiven Druckpulsen z. B. zur extrakorporalen Lithotripsie ist es vorteilhaft eine Quelle, die positive Druckpulse erzeugt, mit einem akustisch harten Reflektor zu versehen. Je nach therapeutischer Zielsetzung können andere Quellen/Reflektorkombinationen sinnvoll sein. Für die extrakorporale Lithotripsie bietet sich ein Messingreflektor in Verbindung mit Wasser als Koppelflüssigkeit an.

Bei der Auswahl des Reflektormaterials ist ferner darauf zu achten, daß außer den bereits genannten Eigenschaften die Ausbreitungsgeschwindigkeit für transversale Oberflächenwellen kleiner oder um geringfügiges größer als die Ausbreitungsgeschwindigkeit der Therapiewellen in der Koppelflüssigkeit ist.

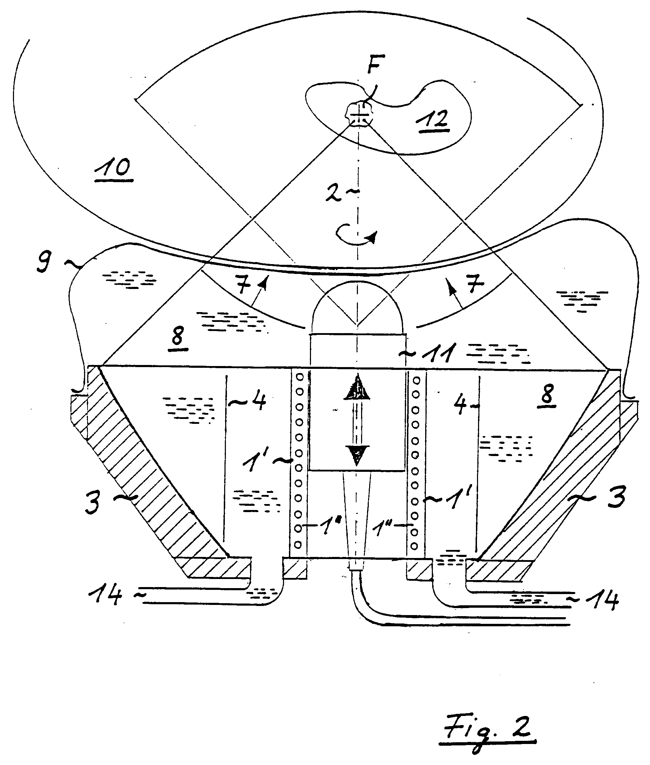

Figur 2 zeigt exemplarisch die in Figur 1 dargestellte Vorrichtung zur Erzeugung von fokussierten akustischen Wellenfeldern komplettiert miz weiteren Funktionselementen zur Verwendung als Lithotripter:

Insbesondere befindet sich bei dem in Figur 2 dargestellten Ausführungsbeispiel innerhalb der zylindrischen Abstrahlfläche 1′ der Schallerzeugungseinheit eine Ultraschallortungseinrichtung 11, z. B. in Form eines Ultraschall-B-Bild-Geräts, die zur Darstellung der Fokuszone F im Körper eines Lebewesens 10 dient.

Weiterhin ist ohne Beschränkung des allgemeinen Erfindungsgedankens die Schallerzeugungseinheit in Figur 2 exemplarisch als elektromagnetisches System dargestellt, das innerhalb der Abstrahlfläche 1 eine Spule 1″ aufweist. Es können selbstverständlich zur Schallerzeugung auch andere Elemente, beispielsweise piezoelektrische Quellen in Zylinderform verwendet werden.

Für die Einkoppelung akustischer Wellen in Körper von Lebewesen ist es vorteilhaft, die Wellen bereits in einem Medium mit gewebeähnlichen akustischen Eigenschaften zu erzeugen, um die Reflexionsverluste gering zu halten. Derartige Medien sind beispielsweise Wasser, Öle oder andere Flüssigkeiten, deren akustischen Impedanzen $\delta * c$ (Dichte * Schallgeschwindigkeit) der Impedanz von lebendem Gewebe im Einkoppelbereich der Therapiewelle nahekommt.

In Figur 2 ist deshalb der Reflektor 3 von einer schalldurchlässigen, akustisch angepaßten Membran 9 abgeschlossen. In dem von dem Reflektor 3 und der Membran 9 gebildeten Raum 8 befindet sich eine Koppelflüssigkeit, so daß sich ein flexibles Kissen ergibt, das unter Vermeidung von Gaseinschlüssen akustisch an den Körper 10 angekoppelt wird.

Die Therapiewellen 4 breiten sich - wie bereits beschrieben - zunächst radial bezüglich der Rotationsachse 2 des Systems aus, und werden anschließend vom Reflektor 3 zum Fokuspunkt F reflektiert. Der akustische Reflektor besitzt dabei die vorstehend beschriebene Form und besteht aus einem Material mit einem hohen Reflexionsgrad in Bezug auf die Koppelflüssigkeit 8.

Das akustische fokussierte Wellenfeld 7 wird über die Membran 9 in den Körper 10 eingekoppelt und mit Hilfe der Ultraschall-Ortungseinheit 11 auf das therapeutische Zielgebiet 12 ausgerichtet. Die Ultraschallsonde ist zur Optimierung der Bildqualität des Ultraschallbildes längs der Achse 2 in Pfeilrichtung verschiebbar angeordnet, so daß sie wahlweise bis an den Körper herangeschoben oder aber zur Vermeidung von Abschattungen des Therapiewellenfeldes zurückgezogen werden kann. Ein entsprechender Positionsmelder sorgt dafür, daß der Fokuspunkt des Therapiefeldes kontinuierlich im Ultraschallbild dargestellt wird. Weiterhin ist die Ultraschallsonde um die Achse 2 drehbar angeordnet, so daß das Ultraschallbild wahlweise verschiedene Schnittebene zur Darstellung bringt. Die Therapieeinheit besitzt neben den notwendigen Versorgungsanschlüssen für die Therapiequelle und die Ultraschallsonde Anschlüsse 14 für einen Volumenausgleich des Koppelkissens durch Zu-bzw. Ableitung geeignet präparierter Koppelflüssigkeiten, so daß eine akustisch günstige Anlegung der Membran 9 an den Körper 10 möglich wird. Die entsprechenden Versorgungsgeräte wie Pumpen, Steuereinheiten, Entgasungsvorrichtungen usw. sind nicht dargestellt.

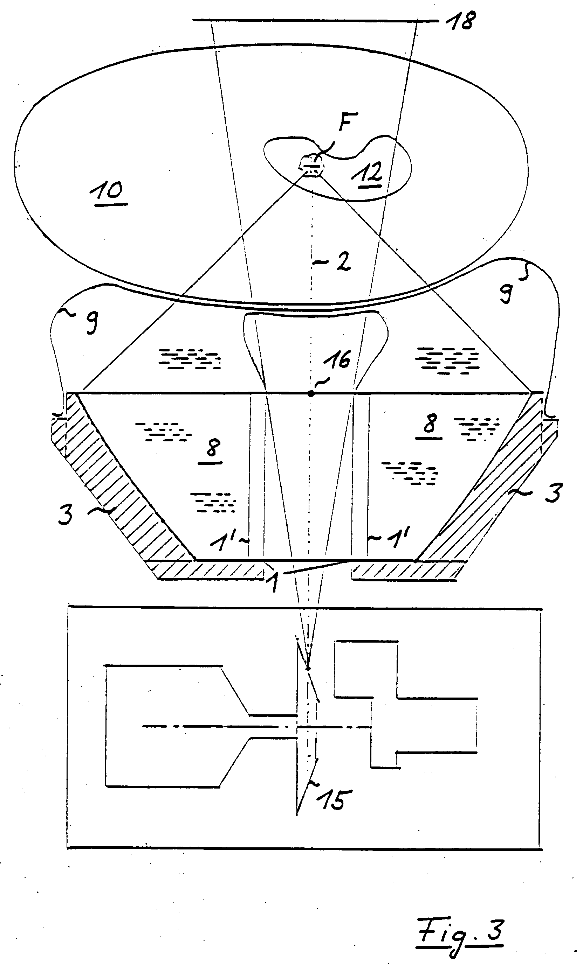

Figur 3 zeigt eine entsprechende Anordnung, bei der die Ultraschallsonde gegen eine Röntgen-Ortungsvorrichtung ausgetauscht ist. Wiederum sind die selben Bezugszeichen wie in den Figuren 1 und 2 verwendet.

Die Röntgenröhre 15 ist dabei so mit der Therapieeinheit verbunden, daß der Fokus der Röntgenröhre 15 auf der Rotationsachse 2 liegt. Für das Ausrichten des Therapiefeldes auf das Zielgebiet 12 ist eine Zielmarkierung 16 auf der Achse 2 angeordnet, die zusammen mit dem Zielgebiet 12 in der Röntgenregistrierebene 18, in der beispielsweise ein Bildverstärker oder ein Röntgenfilm angeordnet ist, abgebildet wird. Eine Justierung des Zielgebietes auf der Achse 2 ist somit möglich. Eine Justierung des Zielgebietes in axialer Richtung auf den Fokuspunkt F kann mittels einer zusätzlichen Röntgenprojektion in geneigter Richtung erfolgen. Die Neigung der Röntgenachse wird dabei so gewählt, daß einerseits das Zielgebiet noch im Röntgenbildfeld verbleibt, ohne daß es durch die Geometrie der Therapiequelle 1 vollständig abgeschattet wird, und andererseits der Fokuspunkt F als Drehzentrum gewählt wird.

Die Zielmarkierung 16 wird in gleicher Weise auf den Zentralstrahl der Röntgenprojektion mitgeschwenkt. Das Zielgebiet ist dann mit dem Therapiefokus zur Deckung gebracht, wenn unter zwei geneigten Röntgenprojektionsrichtungen jeweils das Zielgebiet mit der Zielmarke am selben Ort abgebildet wird.

Eine vorteilhafte Ausführungsform ergibt sich, wenn an Stelle des Schwenks der Röntgenröhre um den Therapiefokus F eine Röhre mit zwei räumlich getrennten Foci in Kombination mit zwei angepaßten Zielmarkierungen verwendet wird.

Weitere vorteilhafte Ausführungsformen ergeben sich, wenn für die räumliche Justierung des Therapiefokus auf das Zielgebiet die beiden unter verschiedenen Projektionswinkeln erzeugten Röntgenbilder mit Hilfe bekannter Darstellungstechniken in Form von stereoskopischen Bildern dargestellt und betrachtet werden.

Zur Verbesserung der Röntgenqualität wird zusätzlich eine Vorrichtung 12 vorgesehen, die z. B. in Form eines aufblasbaren Ballons die Koppelflüssigkeit während der Röntgenbilderzeugung aus dem Röntgenprojektionsstrahlengang verdrängt.

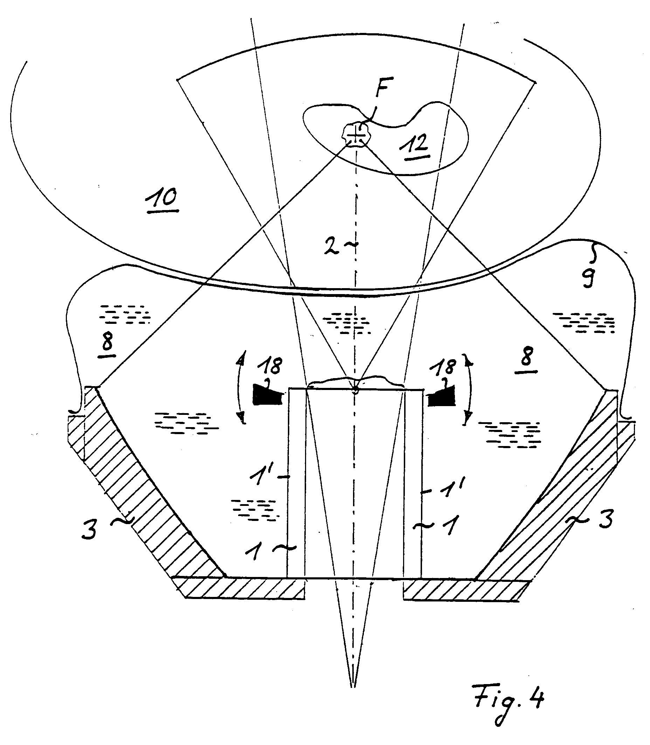

Figur 4 zeigt die Kombination eines erfindungsgemäßen Therapiesystems mit einer Röntgen- und Ultraschallortungseinrichtung derart, daß ein der Geometrie der Therapiequelle 1 angepaßte ringförmige Ultraschallsonde 18 angebracht ist. Entsprechende Ultraschallsonden sind bekannt.

Durch Wobbeln um eine horizontale Drehachse kann nach Art der aus der medizinischen Ultraschalltechnik bekannten Sektorscanner ein B-Bild höchster lateraler Auflösung erzeugt werden. Schließlich kann an Stelle eines mechanischen Schwenks des ringförmigen Ultraschallschwingers 18 ein linear unterteiltes elektronisch angesteuertes ringförmiges Array (annular array) mit großer zentraler Öffnung verwendet werden oder ein radial segmentiertes Array, mit dem durch elektronische Ablenkung des Ultraschallortungsfeldes eine dreidimensionale Scan- und Ortungseinheit realisiert wird.

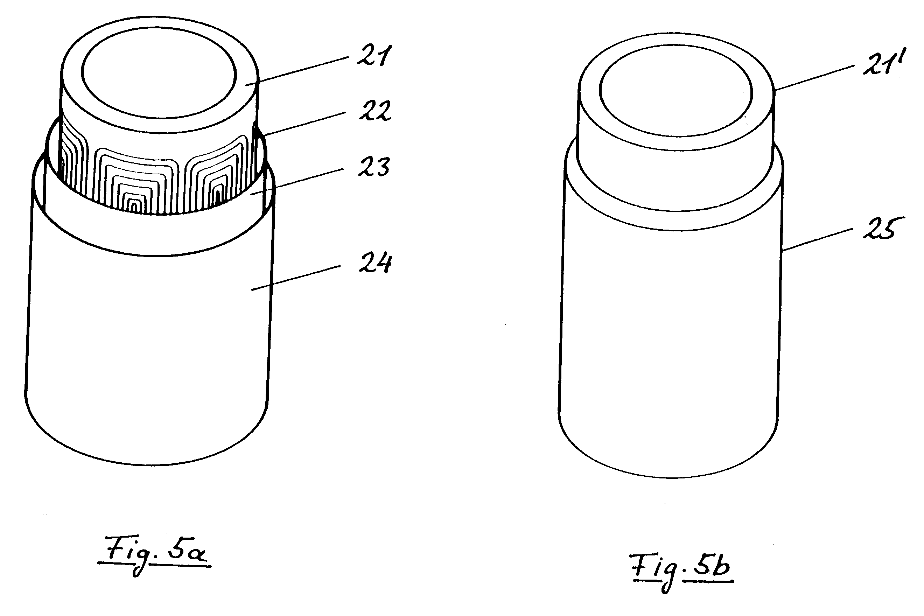

Die Fig. 5a und 5b zeigen Ausführungsformen für die Schallerzeugungseinheit 1. In Fig. 5a ist eine elektromagnetische Pulsschallerzeugungseinheit dargestellt, die einen Spulenträger 21 für eine Spule 22 aufweist. Die Spule 22 besteht zur Energieerhöhung aus mehreren ineinander angeordneten Einzelspulen, die elektrisch parallel geschaltet sind. Weiterhin ist eine Membran 24 und insbesondere eine Metallmembran vorgesehen, die als eigentliche "Schall-abstrahlende Fläche" dient. Zwischen Spule 22 und Membran 24 ist eine Isolierschicht 23 angeordnet, die insbesondere zur Verbesserung der elektrischen Sicherheit beiträgt. Die Membran besteht typischerweise aus einem elektrisch hochleitfähigem Material, wie beispielsweise Gold, Silber, Kupfer oder Legierungen hiervon. Das hochfeste Stützmaterial 21, das elektrisch von der Membran isoliert ist, kann beispielsweise Edelstahl, Titan oder Beryllium sein.

Fig. 5b zeigt eine Variante, bei dem auf einem Träger 21′ ein radial polarisierter Piezokeramikzylinder 25 angeordnet ist. Zusätzlich ist noch eine in der Figur nicht dargestellte Anpaßschicht vorgesehen.

Mit der vorstehend beschriebenen Vorrichtungen lassen sich beispielsweise bei einem Aperturwinkel von 83° und einem Fokusabstand von 15 cm eine Fokuszone von 6 mm lateral und 30 mm axial, d.h. gut an die Fragmentationsanforderungen angepaßte FRÖßen erzielen. Dabei erreichen die Druckamplituden 90 MPa bei Druckanstiegszeiten von ca. 100 ns.

## Patentansprüche

1. Vorrichtung zur Erzeugung von fokussierten akustischen Druckwellen für therapeutische Anwendungen und insbesondere zur Zerstörung von Konkrementen, Körpersteinen etc., mit

   - einer Schallerzeugungseinheit (1), deren rotationssymmetrisch zu einer durch den Fokuspunkt (F) gehenden Achse (2) ausgebildete Mantelfläche die Abstrahlfläche (1') für Druckwellen ist, wobei die Schallerzeugungseinheit (1) in einem Koppelmedium (8) im wesentlichen senkrecht von der durch den Fokuspunkt (F) gehenden Achse (2) axialsymmetrische Druckwellen (4) erzeugt, und

   - einem Reflektor (3), der zur Fokussierung der Druckwellen eine paraboloide Kontur aufweist, dadurch **gekennzeichnet**, daß der Reflektor (3) die Schallerzeugungseinheit (1) umgibt, und daß das von der Schallerzeugungseinheit (1) erzeugte Wellenfeld von besagter Achse (2) weggerichtet ist.

2. Vorrichtung nach Anspruch 1, dadurch **gekennzeichnet**, daß die Schallerzeugungseinheit (1) ein Kreiszylinder oder ein im wesentlichen zylindrischer Kegelstumpf ist.

3. Vorrichtung nach einem der Ansprüche 1 bis 2, dadurch **gekennzeichnet**, daß der Reflektor (3) die Form eines Rotationsparaboloids hat, das die Schallerzeugungseinheit (1) im Bereich der Achse durchsetzt.

4. Vorrichtung nach einem der Ansprüche 1 bis 3, dadurch **gekennzeichnet**, daß die schallerzeugungseinheit (1) eine elektromagnetische Druckwellenerzeugungseinheit ist.

5. Vorrichtung nach einem der Ansprüche 1 bis 3, dadurch **gekennzeichnet**, daß die Schallerzeugungseinheit (1) eine piezoelektrische Druckwellenerzeugungseinheit ist.

6. Vorrichtung nach Anspruch 5, dadurch **gekennzeichnet**, daß auf der Abstrahlfläche (1') Piezo-Schwinger vorgesehen sind.

7. Vorrichtung nach einem der Ansprüche 1 bis 6, dadurch **gekennzeichnet**, daß innerhalb der rotationssymmetrischen Abstrahlfläche (1') der Schallerzeugungseinheit (1) wenigstens eine Ortungseinheit angeordnet ist.

8. Vorrichtung nach Anspruch 7, dadurch **gekennzeichnet**, daß die Ortungseinheit eine Röntgeneinheit (15) ist.

9. Vorrichtung nach Anspruch 7, dadurch **gekennzeichnet**, daß wenigstens eine Ortungseinheit eine Ultraschall-Ortungseinheit (11) ist, die ein sogenanntes B-Bild erzeugt.

10. Vorrichtung nach einem der Ansprüche 7 bis 9, dadurch **gekennzeichnet**, daß die Ortungseinheit längs der Achse (2) verschiebbar und um diese Achse drehbar ist.

11. Vorrichtung nach einem der Ansprüche 1 bis 10, dadurch **gekennzeichnet**, daß der Reflektor (3) derart ausgebildet ist, daß erzeugte Oberflächenwellen nicht mit am Reflektor (3) reflektierten Wellen interferieren.

12. Vorrichtung nach einem der Ansprüche 1 bis 11, dadurch **gekennzeichnet**, daß der Reflektor (3) von einer schalldurchlässig und akustisch angepaßten Membran (9) abgeschlossen ist.

13. Vorrichtung nach einem der Ansprüche 1 bis 12, dadurch **gekennzeichnet**, daß das Koppelmedium (8) in an sich bekannter Weise ein Medium mit gewebeähnlichen akustischen Eigenschaften ist.

14. Vorrichtung nach Anspruch 13, dadurch **gekennzeichnet**, daß das Koppelmedium (8) eine Flüssigkeit ist.

15. Vorrichtung nach einem der Ansprüche 1 bis 14, dadurch **gekennzeichnet**, daß die Schallerzeugungseinheit (1) zur Erzeugung von Schallimpulsen zur therapeutischen Behandlung von biologischem Gewebe einen elektromagnetischen Generator mit einer Spule (22), die von einem Generator impulsförmig erregbar ist, und mit einer schwingfähigen Membran (24) aufweist, die die Ultraschallimpulse abstrahlt, und daß die Spule (22) aus mehreren ineinander angeordneten Einzelspulen besteht, die elektrisch parallel geschaltet und/oder ineinander geschachtelt sind.

16. Vorrichtung nach Anspruch 15, dadurch **gekennzeichnet**, daß zwischen Spule (22) und Membran (24) eine Isolierschicht (23) angeordnet ist.

17. Vorrichtung nach Anspruch 15 oder 16, dadurch **gekennzeichnet**, daß die Membran (24) aus einem elektrisch hochleitfähigem Material besteht, das von einem festen Material gestützt wird.

18. Vorrichtung nach Anspruch 17, dadurch **gekennzeichnet**, daß das feste Stützmaterial (21, 21') elektrisch von der Membran (24) isoliert ist.

19. Vorrichtung nach einem der Ansprüche 16 bis 18, dadurch **gekennzeichnet**, daß das Stützmaterial (21, 21') aus einem Metall- oder einen Kunststoffmaterial besteht.

20. Vorrichtung nach einem der Anspüche 17 bis 19, dadurch **gekennzeichnet**, daß das Stützmaterial (21, 21') magnetisierbar ist.

21. Vorrichtung nach einem der Ansprüche 17 bis 20, dadurch **gekennzeichnet**, daß das Stützmaterial (21, 21') fest mit der Membran (24) verbunden ist.

22. Vorrichtung nach einem der Ansprüche 15 bis 21, dadurch **gekennzeichnet**, daß die Membran (24) und/oder das Stützmaterial (21, 21') keine konstante Dicke haben.

23. Vorrichtung nach einem der Ansprüche 15 bis 22, dadurch **gekennzeichnet**, daß die Membran (24) und/oder das Stützmaterial (21, 21') an die Spule (22) angedrückt sind.

24. Vorrichtung nach einem der Ansprüche 15 bis 23, dadurch **gekennzeichnet**, daß die Membran (24) und/oder das Stützmaterial (21, 21') in ein elastisches Material eingebettet sind.

25. Vorrichtung nach einem der Ansprüche 15 bis 24, dadurch **gekennzeichnet**, daß die Membran (24) eine oder mehrere Sicken aufweist.

**Claims**

1. Device for generating focussed acoustic pressure waves for therapeutical applications, specifically for destroying concrements, corporeal calculi, etc., comprising

   - a sound generator unit (1) whose nappe, which is rotationally symmetrical relative to an axis (2) passing through the focal point (F), is the pressure-wave emitter surface (1'), with said sound generator unit (1) generating axially symmetrical pressure waves in a coupling medium (8), substantially in an orthogonal direction from said axis (2) passing through said focal point (F), and
   - a reflector (3) having a paraboloid shape for focussing said pressure waves, **characterized** in that said reflector (3) surrounds said sound generator unit (1), and that the wave field, which is generated by said sound generator unit (1), is oriented away from said axis (2).

2. Device according to Claim 1, **characterized** in that said sound generator unit (1) is a substantially cylindrical truncated cone.

3. Device according to any of Claims 1 to 2, **characterized** in that said reflector (3) has the shape of a paraboloid of revolution, which passes through said sound generator unit (1) in the region of said axis.

**4.** Device according to any of Claims 1 to 3, **characterized** in that said sound generator unit (1) is an electromagnetic unit generating pressure waves.

**5.** Device according to any of Claims 1 to 3, **characterized** in that said sound generator unit (1) is a piezoelectric unit generating pressure waves.

**6.** Device according to Claim 5, **characterized** in that piezoelectric oscillators are provided on said emitter surface (1').

**7.** Device according to any of Claims 1 to 6, **characterized** in that at least one locating unit is disposed inside said rotationally symmetrical emitter surface (1') of said sound generator unit (1).

**8.** Device according to Claim 7, **characterized** in that said locating unit is an X-ray unit.

**9.** Device according to Claim 7, **characterized** in that at least one locating unit is an ultrasonic locating unit (11) which generates a so-called B-image.

**10.** Device according to any of Claims 7 to 9, **characterized** in that said locating unit is adapted for displacement along said axis (2) and for pivoting about this axis.

**11.** Device according to any of Claims 1 to 10, **characterized** in that said reflector (3) is so designed that surface waves will not interfere with waves reflected at said reflector (3).

**12.** Device according to any of Claims 1 to 11, **characterized** in that said reflector (3) is sealed by a membrane adapted in terms of sonic transmission and acoustics.

**13.** Device according to any of Claims 1 to 12, **characterized** in that said coupling medium (8) is a medium having acoustic characteristics similar to those of tissue, in a manner known per se.

**14.** Device according to Claim 13, **characterized** in that said coupling medium is a liquid.

**15.** Device according to any of Claims 1 to 14, **characterized** in that said sound generator unit (1) for generating sonic pulses for the therapeutic treatment of biological tissue comprises an electromagnetic generator having a coil (22) adapted for pulsed excitation by a generator, and having a membrane able to oscillate (24), which emits the ultrasonic pulses, and that said coil (22) consists of a plurality of individual coils disposed in one another, which are connected in an electrical parallel circuit and/or which are mutually nested.

**16.** Device according to Claim 15, **characterized** in that an insulating layer (23) is provided between said coil (22) and said membrane (24).

**17.** Device according to Claim 15 or 16, **characterized** in that said membrane (24) is made of a highly conductive material which is supported by a firm material.

**18.** Device according to Claim 17, **characterized** in that said firm material (21, 21') is electrically insulated from said membrane (24).

**19.** Device according to any of Claims 16 to 18, **characterized** in that said supporting material (21, 21') consists of a metallic or synthetic material.

**20.** Device according to any of Claims 17 to 19, **characterized** in that said supporting material (21, 21') is magnetizable.

**21.** Generator according to any of Claims 17 to 20, **characterized** in that said supporting material (21, 21') is fixedly connected to said membrane (24).

**22.** Generator according to any of Claims 15 to 21, **characterized** in that said membrane (24) and/or said supporting material (21, 21') do not have a constant thickness.

**23.** Generator according to any of Claims 15 to 22, **characterized** in that said membrane (24) and/or said supporting material (21, 21') are pressed against said coil (22).

**24.** Generator according to any of Claims 15 to 23, **characterized** in that said membrane (24) and/or said supporting material (21, 21') are embedded in a resilient material.

**25.** Generator according to any of Claims 15 to 24, **characterized** in that said membrane (24) comprises one or several beads.

**Revendications**

**1.** Dispositif à générer des ondes de pression acoustiques focalisées à des fins thérapeutiques, en particulier à détruire des concrétions, calculs dans le corps, etc., comprenant

- une unité génératrice sonore (1), dont la nappe, qui est configurée à symétrie de révolution relative à l'axe (2) passant par le point focal (F), est la surface émettrice (1'), qui émet des ondes de pression, ladite unité génératrice sonore (1) engendrant des ondes de pression axisymétriques dans un milieu de couplage (8), essentiellement en sens orthogonal à partir dudit axe (2) passant par ledit point focal (F), et

- un réflecteur (3) à contour paraboloïde à focaliser lesdites ondes de pression,
  **caractérisé** en ce que ledit réflecteur (3) entoure ladite unité génératrice sonore (1), et en ce que le champ d'ondes, qui est engendré par ladite unité génératrice sonore (1), est dirigé de façon à s'étendre à partir dudit axe (2).

2. Dispositif selon la revendication 1,
   **caractérisé** en ce que ladite unité génératrice sonore (1) est un tronc de cône essentiellement cylindrique.

3. Dispositif selon une quelconque des revendications 1 à 2,
   **caractérisé** en ce que ledit réflecteur (3) présente la forme d'un paraboloïde de révolution, qui s'étend à travers ladite unité génératrice sonore (1) dans la zone dudit axe.

4. Dispositif selon une quelconque des revendications 1 à 3,
   **caractérisé** en ce que ladite unité génératrice sonore (1) est une unité génératrice électromagnétique à engendrer des ondes de pression.

5. Dispositif selon une quelconque des revendications 1 à 3,
   **caractérisé** en ce que ladite unité génératrice sonore (1) est une unité génératrice piézoélectrique à engendrer des ondes de pression.

6. Dispositif selon la revendication 5,
   **caractérisé** en ce que des transducteurs piézoélectriques sont prévus sur ladite surface émettrice (1').

7. Dispositif selon une quelconque des revendications 1 à 6,
   **caractérisé** en ce qu'au moins une unité de repérage est disposée à l'intérieur de ladite surface émettrice (1') à symétrie de révolution de ladite unité génératrice sonore (1).

8. Dispositif selon la revendication 7,
   **caractérisé** en ce que ladite unité de repérage est une unité radiographique.

9. Dispositif selon la revendication 7,
   **caractérisé** en ce qu'au moins une unité de repérage est une unité de repérage par ultra-sons (11) qui engendre une image dite B.

10. Dispositif selon une quelconque des revendications 7 à 9,
    **caractérisé** en ce que ladite unité de repérage est déplaçable le long dudit axe (2) et pivotant autour de cet axe.

11. Dispositif selon une quelconque des revendications 1 à 10,
    **caractérisé** en ce que ledit réflecteur (3) est configuré de façon qu'il n'y ait pas une interférence entre des ondes de surface et des ondes réfléchies audit réflecteur (3).

12. Dispositif selon une quelconque des revendications 1 à 11,
    **caractérisé** en ce que ledit réflecteur (3) est fermé par une membrane adaptée en vue de la transmission du son et de l'acoustique.

13. Dispositif selon une quelconque des revendications 1 à 12,
    **caractérisé** en ce que ledit milieu de couplage (8) est un milieu qui présente des caractéristiques acoustiques similaires à celles des tissus, d'une façon connue en soi.

14. Dispositif selon la revendication 13,
    **caractérisé** en ce que ledit milieu de couplage est un liquide.

15. Dispositif selon une quelconque des revendications 1 à 14,
    **caractérisé** en ce que ladite unité génératrice sonore (1) à engendrer des impulsions sonores pour le traitement thérapeutique des tissus biologiques comprend un générateur électromagnétique à une bobine (22) adaptée à être excité à impulsions par un générateur, et à une membrane capable de résonner (24), qui émet les impulsions d'ultra-sons, et
    en ce que ladite bobine (22) est constituée par une pluralité des bobines individuelles disposées l'une dans l'autre, qui se trouvent en montage électrique en parallèle et/ou sont intercalées l'une dans l'autre.

16. Dispositif selon la revendication 15,
    **caractérisé** en ce qu'une couche isolante (23) est disposée entre ladite bobine (22) et ladite membrane (24).

17. Dispositif selon la revendication 15 ou 16,
    **caractérisé** en ce que ladite membrane (24) est faite d'un matériau à haute conductivité, qui est soutenu par un matériau résistant.

18. Dispositif selon la revendication 17,
    **caractérisé** en ce que ledit matériau résistant (21, 21') est électriquement isolé de ladite membrane (24).

19. Dispositif selon une quelconque des revendications 16 à 18,
**caractérisé** en ce que ledit matériau de support (21, 21') est fait d'un matériau métallique ou synthétique.

20. Dispositif selon une quelconque des revendications 17 à 19,
**caractérisé** en ce que ledit matériau de support (21, 21') est magnétisable.

21. Dispositif selon une quelconque des revendications 17 à 20,
**caractérisé** en ce que ledit matériau de support (21, 21') est fixé à ladite membrane (24).

22. Dispositif selon une quelconque des revendications 15 à 21,
**caractérisé** en ce que ladite membrane (24) et/ou ledit matériau de support (21, 21') ne présentent pas une épaisseur constante.

23. Dispositif selon une quelconque des revendications 15 à 22,
**caractérisé** en ce que ladite membrane (24) et/ou ledit matériau de support (21, 21') sont pressés contre ladite bobine (22).

24. Dispositif selon une quelconque des revendications 15 à 23,
**caractérisé** en ce que ladite membrane (24) et/ou ledit matériau de support (21, 21') sont noyés dans un matériau élastique.

25. Dispositif selon une quelconque des revendications 15 à 24,
**caractérisé** en ce que ladite membrane (24) comprend une ou plusieurs moulures.

$$y^2 = 2px$$

Fig.1

EP 0 369 177 B1

Fig. 2

*Fig. 3*

Fig. 4

Fig. 5b

Fig. 5a